Europäisches Patentamt
European Patent Office
Office européen des brevets

(19) (11) Numéro de publication: **0 170 590**
**A1**

(12) # DEMANDE DE BREVET EUROPEEN

(21) Numéro de dépôt: 85401501.3

(22) Date de dépôt: 22.07.85

(51) Int. Cl.[4]: C 07 D 209/48
C 08 G 73/10, C 09 D 3/49
C 09 J 3/16

(30) Priorité: 25.07.84 FR 8411841

(43) Date de publication de la demande: 05.02.86 Bulletin 86/6

(84) Etats contractants désignés: BE DE GB IT NL

(71) Demandeur: CENTRE D'ETUDE DES MATERIAUX ORGANIQUES POUR TECHNOLOGIES AVANCEES
Autoroute Lyon-Vienne Echangeur de Solaize Vernaison Solaize
F-69390 Vernaison(FR)

(72) Inventeur: Malinge, Jean
6, Allée Ho Chi Minh
F-69700 Givors(FR)

(72) Inventeur: Rabilloud, Guy
27, Boulevard Clémenceau
F-38100 Grenoble(FR)

(72) Inventeur: Sillion, Bernard
93, rue Juliot Curie
F-69005 Lyon(FR)

(74) Mandataire: Colas des Francs, Jean et al,
Institut Français du Pétrole 4, Avenue de Bois Préau
F-92502 Rueil-Malmaison(FR)

(54) **Nouveaux précurseurs de réseaux de copolyphtalocyanine-imides, leur procédé de préparation et les réseaux qui en derivent.**

(57) On décrit des compositions de polyimides aromatiques, précurseurs de copolyphtalocyanine-imides, leur procédé de préparation et les copolyphtalocyanine-imides qui en dérivent.

Ces compositions peuvent résulter de la réaction à 80 - 300° C d'un composé aromatique tétraacide, diester-diacide, tétraester ou dianhydride, de formule

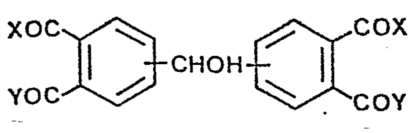

avec un amino-dinitrile aromatique 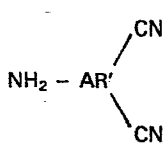

et éventuellement une diamine aromatique $NH_2 - Ar - NH_2$.

Par un chauffage ultérieur à 200 - 300° C, on obtient, par réticulation, des copolyphtalocyanine-imides, qui sont des résines durcies, stables à 250 - 300° C en service continu.

EP 0 170 590 A1

Croydon Printing Company Ltd.

La présente invention a pour objet de nouvelles compositions d'oligomères imides thermodurcissables présentant une solubilité élevée dans les solvants organiques. Elle concerne plus particulièrement les compositions d'oligomères formés de cycles imides reliés entre eux par des groupements benzhydrol et terminés par des groupes ortho-dinitriles.

L'invention concerne le procédé de cyano-addition des groupes ortho-dinitriles conduisant à des polyphtalocyanines par un phénomène d'oxydoréduction initié par les groupements benzhydrols.

L'invention concerne également les réseaux de polyimides-copolyphtalocyanines qui sont formés au cours des réactions de cyano-addition.

Les compositions de polyimides solubles précurseurs des copolyphtalocyanines-imides peuvent être employées comme liants dans la fabrication de matériaux composites, comme adhésifs, comme films et vernis isolants et comme matières premières pour faire des objets moulés et des matériaux cellulaires.

Les phtalocyanines simples sont en général des composés solides de couleur vert sombre, avec une température de fusion élevée et une très faible solubilité dans les solvants organiques. Il est donc extrêmement difficile de préparer des polyphtalocyanines de haute masse moléculaire ou d'utiliser ce type de polymères sous leur

forme cyclisée finale. Par contre, les réactions de cyano-addition peuvent être utilisées pour réticuler des monomères ou des oligomères fusibles et solubles, les réactions de réticulation intervenant par simple chauffage au moment de la mise en oeuvre des produits.

De telles réactions de cyano-addition ont été décrites avec des composés aromatiques ou arylaliphatiques contenant des enchaînements amides, azométhines ou éthers (Walton et Griffith : Applied Polymer Symposium, 1975, 26, 429 ; Polymer Science and Technology, 1975, 9B, 665 ; A.C.S. Division of Organic Coatings and Plastics Chemistry, 1978, 38, 596. Keller et coll., SAMPE Quaterly, juillet 1981, p. 1). Dans ces publications et dans les brevets US n° 4 056 560, 4 057 569, 4 102 873, 4 116 945, 4 136 107, 4 209 458, 4 223 123, 4 234 712 et 4 238 601, il est indiqué que la formation des phtalocyanines est une réaction très lente qui, en l'absence de sels métalliques, peut durer plusieurs heures et parfois plusieurs jours à une température généralement comprise entre 200 et 300°C. De plus, Marullo et Snow (ACS Symposium series, vol. 195, 1982, p. 325) ont montré que la sélectivité de la réaction de formation des phtalocyanines est très faible et que le réseau réticulé est en fait un mélange complexe contenant plusieurs types de systèmes hétérocycliques. Enfin, la stabilité thermique des résines thermodurcies est limitée par la nature des enchaînements qui constituent les molécules de départ.

Pour accroître la stabilité thermique des résines de phtalocyanines, on peut introduire dans la chaîne macromoléculaire des structures hétérocycliques très thermostables comme par exemple des imides aromatiques. Mais les polyimides, même sous forme d'oligomères de faible masse moléculaire, ont une température de fusion trop élevée et une solubilité trop faible pour constituer de bons précurseurs de polyphtalocyanines.

On a découvert, et c'est l'un des objets de l'invention, que des oligomères contenant des cycles imides aromatiques reliés par des enchaînements benzhydrols constituaient des compositions assez exceptionnelles pour préparer des résines fusibles, solubles et thermodurcissables par des réactions ultérieures de cyano-addition. Le premier avantage de ces compositions est une solubilité particulièrement

élevée dans de nombreux solvants organiques polaires, ce qui permet de préparer des solutions ayant une forte teneur en matière sèche. Le second avantage tient à la remarquable stabilité thermique de ce type de polyimides qui donnent, après réaction de cyano-addition, des résines utilisables entre 250 et 300°C en service continu. Enfin, et c'est l'un des résultats les plus intéressants observés avec ces compositions, leur temps de gel au moment de la réticulation thermique est beaucoup plus court que celui qui est généralement décrit pour les autres polyphtalocyanines. Ce comportement tout à fait surprenant qui est un des objets de l'invention est dû à un phénomène d'oxydoréduction intramoléculaire intervenant entre le groupement benzhydrol et les groupes ortho-dinitriles comme le démontrent les exemples comparatifs auxquels il a été procédé.

Cette réaction se traduit par un temps de gel de quelques minutes seulement à une température comprise entre 180 et 300°C. Les cycles de fabrication des matériaux sous presse ou dans un autoclave sont donc considérablement raccourcis si on les compare à tous les autres polymères faisant appel aux réactions de cyano-addition.

L'invention a plus particulièrement pour objet des compositions de résines de polyimides précurseurs de réseaux de copolyphtalocyanines-imides de formule générale :

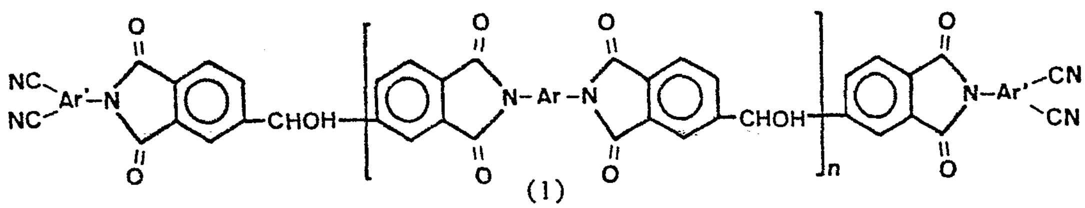

(1)

Dans ces formules, le radical Ar est un radical aromatique divalent carbocyclique ou hétérocyclique, dont les deux valences sont sur des atomes de carbone distincts non situés en position ortho l'un par rapport à l'autre. Le radical Ar peut être formé d'un cycle ou de plusieurs cycles qui sont alors accolés ou reliés entre eux, chaque cycle étant formé de préférence de 5 à 7 atomes dont une partie peut consister en atomes d'oxygène, de soufre et/ou d'azote.

Lorsque le radical Ar comporte plusieurs cycles reliés entre eux, les éléments de liaison sont par exemple la liaison simple

ou l'un des atomes et groupements suivants :
$-O-$ ; $-S-$ ; $-SO-$ ; $-SO_2-$ ; $-CH_2-$ ; $CF_2-$ ; $-C(CH_3)_2-$ ; $-CO-$ ; $-CHOH-$ ; $-COO-$ ; $-CONH-$.

Le radical Ar' est un radical aromatique trivalent carbocyclique ou hétérocyclique, dont les trois valences sont sur des atomes de carbone distincts, deux d'entre eux, ceux qui portent les groupes nitriles étant obligatoirement situés en position ortho l'un par rapport à l'autre. Le radical Ar' peut être formé d'un cycle ou de plusieurs cycles accolés ou reliés entre eux comme défini précédemment pour le radical Ar.

n est un nombre qui peut prendre toutes les valeurs de 0 à 50 et qui indique le degré de polycondensation. Le nombre n n'est pas accessible directement, mais sa valeur moyenne se déduit des proportions molaires des réactifs utilisées pour préparer les compositions d'oligomères benzhydrolimides. La détermination du nombre n est explicitée ci-après avec la technique de préparation des compositions de polyimides. Lorsque le nombre n est nul, les produits obtenus peuvent être représentés par la formule générale

(2)
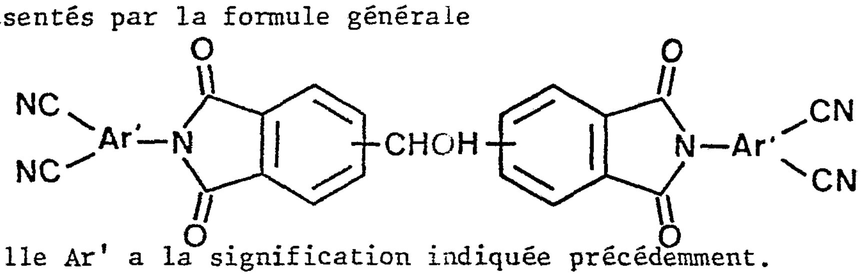

dans laquelle Ar' a la signification indiquée précédemment.

Les compositions de polyimides de formule (1) peuvent être préparées en faisant réagir au moins un composé aromatique (A) de formule générale

(3)
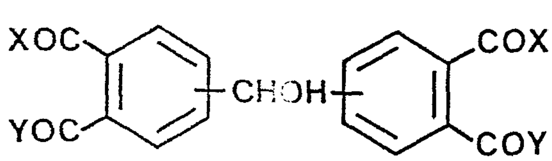

avec au moins un amino-dinitrile aromatique (C) de formule générale

(5)
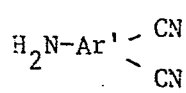

et facultativement

avec au moins une diamine aromatique (B) de formule générale

(4) $H_2N-Ar-NH_2$

Dans ces formules Ar et Ar' ont la signification indiquée précédemment. X et Y, identiques ou différents, sont des radicaux qui représentent la nature des centres réactifs, lesquels peuvent être des acides carboxyliques, leurs esters ou leurs anhydrides. Lorsque X et Y sont des radicaux hydroxyles, le composé (A) est l'acide benzhydrol tétracarboxylique-3,3',4,4'. Le dianhydride de cet acide est représenté par la formule (3) dans laquelle X et Y sont confondus et représentent un atome d'oxygène.

Lorsque X est un radical hydroxyle et Y un radical alcoxy renfermant de préférence de 1 à 13 atomes de carbone, le composé de formule (3) représente un bis-orthoacide-ester (ou diester) de l'acide benzhydrol tétracarboxylique. Enfin, si X et Y sont tous deux des radicaux hydroxycarbyloxy, le produit est un tétraester d'alkyle de ce même acide.

L'enchaînement carbinol qui sépare les deux cycles aromatiques du benzhydrol est placé au milieu de ces cycles pour indiquer les possibilités d'isomérie dans le cas des dérivés dissymétriques.

Parmi les diamines qui conviennent pour la présente invention, on peut citer les diamino-1,3 et 1,4 benzène, les diamino-3,3' et 4,4' diphényl méthane, les diamino-3,3' et 4,4' diphényl éther, la benzidine, les diamino-3,3' et 4,4' diphényl sulfure, les diamino-3,3' et 4,4' diphényl sulfone, les diamino-3,3' et 4,4' benzophénone, les diamino-3,3' et 4,4' benzhydrol, les diamino-3,3' et 4,4' benzanilide, les diamino-3,3' et 4,4' benzoate de phényle, le bis(amino-4 phényl) diméthyl silane, les diamino-2,4, (2,6) et 3,5 pyridine, la diméthoxy-3,3' benzidine.

Parmi les amino-dinitriles aromatiques utilisables dans le cadre de l'invention, on peut citer les amino-3 et 4 phtalonitrile, les para et méta aminophénoxy-4 phtalonitrile, l'amino-4' dicyano-3,4 biphényle, l'amino-4' dicyano-3,4 diphényl méthane, les amino-3' et 4' dicyano-3,4 benzophénone, les amino-3' et 4' dicyano-3,4 benzhydrol.

Parmi les dérivés de l'acide benzhydrol tétracarboxylique-3,3',4,4' qui conviennent pour la présente invention, on peut citer le tétra-acide lui-même, son dianhydride, les diesters de méthyle, d'éthyle, d'isopropyle, de butyle et d'hydroxy-2 éthyle, les tétra-

esters de méthyle et d'éthyle.

Les résines de formule générale (1) peuvent être préparées en une ou deux étapes selon que tous les réactifs sont introduits simultanément ou en deux fois. Dans les deux cas, la stoechiométrie globale de la réaction est la même, car elle consiste à engager dans la condensation un nombre de fonctions amines, provenant de la diamine aromatique et de l'amino-dinitrile, sensiblement égal au nombre de fonctions réactives du composé de formule (3). Plus précisément, pour un centre réactif ortho-difonctionnel de ce dernier composé, on utilise de préférence au total de 0,9 à 1,1 fonction amine. Les meilleurs résultats sont obtenus avec une proportion comprise entre 0,98 et 1,02 fonction amine-primaire.

Comme il a été dit précédemment, les fonctions amines primaires proviennent soit en totalité de l'aminodinitrile de formule (5) ; le degré de polycondensation n est alors égal à zéro ; soit en partie de l'aminodinitrile et en partie de la ou des diamines aromatiques de formule (4) ; le degré de polycondensation n est alors plus grand que zéro.

Les proportions respectives des deux composés aminés fixent le degré de polycondensation puisque la masse moléculaire de la composition de résine de polyimide est d'autant plus élevée que le rapport molaire entre le composé (A) et la diamine aromatique se rapproche de l'unité.

Selon les applications envisagées, le nombre n peut prendre toutes les valeurs de 0 à 50 et de préférence entre 0 et 20. Cela signifie que pour 1 mole de composé (A) on peut par exemple employer de 0 à 0,98 et de préférence de 0 à 0,95 mole de diamine aromatique. Comme il y a au départ deux centres ortho-difonctionnels réactifs par mole de composé (A) auxquels on oppose de 0 à 1,96 (de préférence de 0 à 1,90) fonctions amine provenant de la diamine, il faut ajouter pour rétablir l'équilibre stoechiométrique respectivement de 2 à 0,04 moles et de préférence de 2 à 0,1 mole d'amino-dinitrile.

Les valeurs indiquées ci-dessus sont données à titre d'exemple pour montrer comment il est possible de relier le degré de polycondensation des résines de formule générale (1) aux proportions

relatives des divers réactifs.

Les résines de formule générale (1) où n est différent de zéro peuvent être préparées en deux étapes successives qui sont effectuées dans le même réacteur. Au cours de la première étape, la diamine aromatique de formule (4) (ou un mélange de diamines aromatiques) est mise en réaction avec un excès de composé aromatique de formule (3) dans un solvant organique qui est chauffé à une température suffisante pour promouvoir la réaction de polycondensation et la réaction de cyclisation en imide de tous les groupes réactifs antagonistes.

Les compositions obtenues à la fin de la première étape sont formées d'oligomères statistiquement terminés par les groupes réactifs du composé utilisé en excès. Ces oligomères de benzhydrolimides peuvent être représentés par la formule générale

(6) 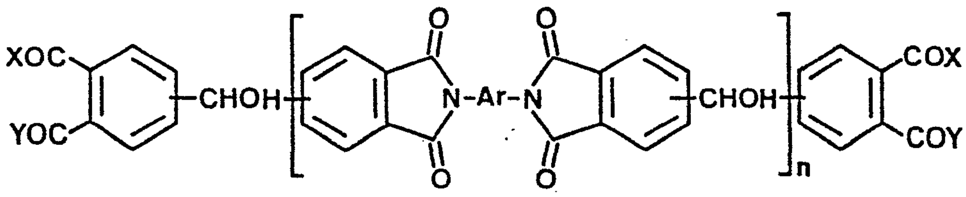

dans laquelle Ar, X, Y et n ont la signification indiquée précédemment. Ils présentent en général une masse moléculaire moyenne de 800 à 25000.

A la fin de cette première étape, l'amino-dinitrile de formule (5) est introduit dans le milieu réactionnel en quantité suffisante pour apporter au moins une fonction amine pour chaque centre réactif ortho-difonctionnel du mélange d'oligomères imides de formule (6). La réaction de condensation est alors poursuivie jusqu'à ce qu'il n'y ait pratiquement plus de fonctions amines libres dans le milieu réactionnel.

Les résines de formule générale (1) peuvent également être préparées en une seule étape en mélangeant dans un solvant organique simultanément les trois réactifs (A), (B) et (C) et en chauffant ce mélange jusqu'à ce que la réaction de polycondensation soit terminée. Les proportions de chaque réactif sont, comme il a été indiqué plus haut, calculées en fonction de la masse moléculaire moyenne désirée.

Les compositions de polyimides de faible masse moléculaire de formule (2) sont préparées par le même procédé en faisant réagir dans un solvant organique un équivalent molaire de composé aromatique de formule (3) avec au moins deux équivalents d'amino-dinitrile de

formule (5).

Les solvants utilisables avec lesquels on prépare les compositions de l'invention sont des composés organiques polaires, c'est à dire renfermant un ou plusieurs hétéroatomes tels que O, N, S, P, Cl, inertes vis à vis des monomères et des polymères. Parmi ces solvants, on peut citer le phénol, les crésols, les xylénols, le chlorobenzène, les dichlorobenzènes, les mono et diéthers de l'éthylèneglycol et du diéthylèneglycol, le diméthylformamide, le diméthylacétamide, l'hexaméthylphosphotriamide, la tétraméthyl urée, le diméthylsulfoxyde, la pyridine, la quinoléine, la N-méthylpyrrolidone, le dioxanne, le tétrahydrofuranne, la tétraméthylènesulfone.

Ces solvants peuvent être utilisés seuls ou mélanges entre eux, ou encore en mélange avec d'autres composés organiques liquides comme les hydrocarbures aromatiques, les alcools, les cétones, les esters ou les composés halogénés.

La concentration initiale des monomères dans le solvant de réaction n'est pas critique mais elle est généralement comprise entre 20 et 80 % en poids. En fin de réaction, la concentration de la composition résineuse est ajustée à une valeur telle que la solution ait une viscosité dynamique bien adaptée à l'utilisation envisagée. En d'autres termes, la concentration finale dépend de la masse moléculaire des oligomères imides, de la nature du ou des solvants et de la température de mise en oeuvre. Pour certaines applications où la résine de polyimides doit être utilisée sous forme solide ou en solution dans un autre solvant que celui qui a servi à la préparer, le produit est précipité dans un composé liquide non-solvant. Pour des raisons de commodité, cette précipitation est généralement effectuée dans l'eau si le solvant de base est miscible à l'eau. Parmi les autres non-solvants, on peut citer les alcools, l'éther éthylique et les hydrocarbures aliphatiques.

La température de la réaction de polycondensation peut varier dans un très large domaine, entre 80 et 300°C. Elle est généralement fixée à une valeur telle que la réaction des fonctions amines sur les dérivés de l'acide benzhydroltétracarboxylique s'effectue à une vitesse raisonnable. Selon le solvant et les réactifs utilisés,

une température comprise entre 100 et 200°C donne généralement de bons résultats. Au-dessus de 200°C, les réactions de formation du réseau réticulé peuvent perturber le cycle normal de la polycondensation linéaire. Les produits volatils formés en cours de réaction peuvent être laissés dans le milieu, mais ils sont généralement éliminés par distillation au fur et à mesure de leur formation, ce qui permet de contrôler l'avancement de la réaction.

La formation des réseaux phtalocyanine à partir des résines précurseurs de l'invention est réalisée au moment de la mise en oeuvre. Les propriétés finales de la résine thermodurcie dépendent étroitement de la nature de la diamine utilisée et de la longueur des chaînes de polyimides entre chaque groupe ortho-dinitrile.

Les résines précurseurs de co-polyphtalocyanines-imides de faible masse moléculaire ont une température de fusion qui est généralement comprise entre 150 et 300°C. Au-delà d'une certaine longueur de chaîne, c'est à dire pour un degré de polycondensation supérieur à 7 ou 8, les résines de polyimides ont une température de transition vitreuse qui se situe entre 220 et 350°C selon la nature de la diamine utilisée.

Le choix de la ou des compositions de résines de polyimides utilisables pour une application déterminée dépend d'une part des propriétés recherchées et d'autre part des techniques de mise en oeuvre imposées par le type d'outillage disponible. Comme les résines de différentes masses moléculaires sont parfaitement compatibles entre elles, il est possible d'utiliser des mélanges en proportions variées de résines fusibles de faible masse moléculaire avec des résines de masse moléculaire plus élevée.

Au moment de la mise en oeuvre, les résines de polyimides thermodurcissables de l'invention sont chauffées à une température suffisante pour promouvoir les réactions de cyano-addition des groupes ortho-dinitriles. La formation des cycles phtalocyanines se traduit par le développement d'une coloration verte qui devient de plus en plus foncée lorsque la polymérisation avance. En spectroscopie infrarouge, les bandes d'absorption des groupes nitriles à 2230 $cm^{-1}$ disparaissent progressivement.

Avec les compositions de polyimides de faible masse moléculaire dont la température de fusion est par exemple comprise entre 150 et 300°C, les réactions de réticulation sont avantageusement conduites entre 200 et 300°C. La viscosité du mélange fondu augmente rapidement et la gélification intervient après 5 à 15 minutes de chauffage. La polymérisation se poursuit ensuite plus lentement en phase solide jusqu'à ce que tous les groupes nitriles aient pratiquement réagi. En admettant que la formation des cycles phtalocyanines est la réaction prédominante de cyano-addition, les compositions de polyimides réticulés répondent à la formule générale

(7) 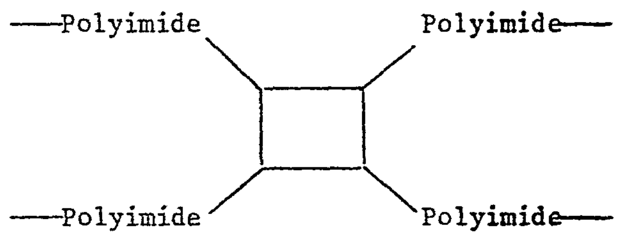

dans laquelle le cycle central représente la 29H, 31H-phtalocyanine de formule

(8) 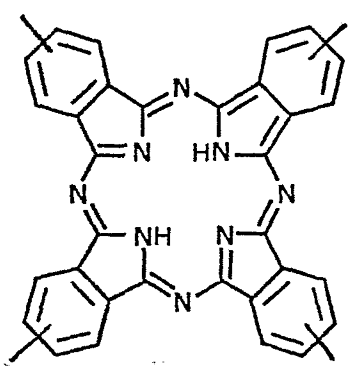

et "polyimide" indique un enchaînement de formule générale

(9) 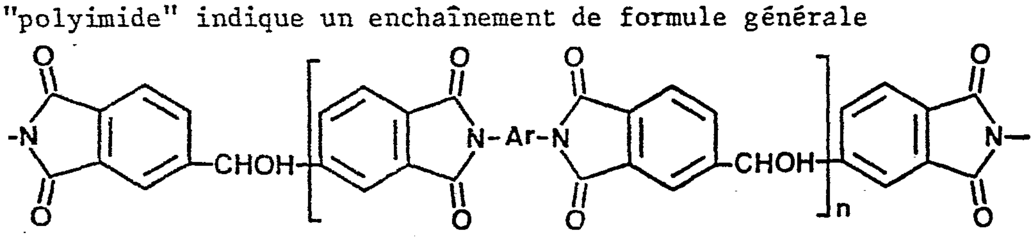

dans laquelle Ar et n ont la signification indiquée précédemment.

L'invention sera décrite de façon plus précise en liaison avec les exemples spécifiques ci-après dans lesquels les détails sont donnés à titre illustratif et non limitatif. Dans ces exemples, les réactions de polycondensation sont effectuées sous agitation et en atmosphère inerte pour éviter l'oxydation des amines aromatiques.

Dans ces exemples, les viscosités inhérentes, lorsqu'on les donne, sont mesurées à 30°C pour une concentration de 5 grammes de résine de polyimides dans 1 litre de N-méthylpyrrolidone. Les masses moléculaires, lorsqu'elles sont indiquées, correspondent à des masses moléculaires moyennes en nombre calculées d'après les proportions relatives des produits de départ.

Pour simplifier l'écriture des noms de produits chimiques les mélanges de composés isomères sont désignés par un terme au singulier comme par exemple le diester de méthyle de l'acide benzhydrol tétracarboxylique-3,3',4,4'.

Deux composés ne contenant pas d'enchaînements benzhydrol sont décrits dans les exemples A et B. Ces produits, le benzoyl-4 (dicyano-3,4 phényl)-N phtalimide et le bis-(dicyano-3,4 phényl)-N,N' carbonyldiphtalimide-4,4' ont été préparés à titre comparatif pour montrer que le phénomène d'oxydoréduction intramoléculaire est réellement dû à la présence des enchaînements benzhydrol dans les résines de polyimides précurseurs de co-polyphtalocyanine-imides.

Le composé modèle de l'exemple C, le (dicyano-3,4 phényl)-N hydroxy-αbenzyl-4 phtalimide a été également préparé pour mettre en évidence cette réaction d'oxydoréduction.

Exemple A (comparatif)

Un mélange de 5,044 g d'anhydride benzoyl-4 phtalique et de 2,863 g d'amino-4 phtalonitrile est mis en réaction à 20°C dans 15 cm3 de N-méthylpyrrolidone (NMP). Au bout de 2 heures, la solution est chauffée à 160°C pendant 7 heures en distillant l'eau formée. Le produit est précipité dans le dichlorométhane. Après séchage, le rendement est de 7,1 g en produit brut qui est purifié par chromatographie en phase liquide sur une colonne de silice. Le benzoyl-4 (dicyano-3,4 phényl)-N phtalimide a une température de fusion proche de 250°C.

Exemple B (comparatif)

Dans des conditions identiques à celles de l'exemple A, on fait réagir 3,222 g d'anhydride benzophénone tétracarboxylique-3,3', 4,4' et 2,863 g d'amino-4 phtalonitrile dans 15 cm3 de NMP. On obtient 5,5 g de bis-(dicyano-3,4 phényl)-N,N' carbonyldiphtalimide-4,4' ayant un point de fusion de 260°C.

Exemple C

Un mélange de 115 g d'anhydride benzoyl-4 phtalique et de 300 cm3 de méthanol est chauffé pendant 2 heures à 65°C. Après refroidissement, on ajoute 6 g de palladium sur charbon et 50 cm3 de méthanol. La fonction cétone est alors hydrogénée à température ambiante sous une pression d'hydrogène de 10 bars pendant 30 minutes. Le catalyseur est éliminé par filtration et le solvant distillé sous vide. Le rendement en monoester méthylique de l'acide benzhydrol dicarboxylique-3,4 est de 120 g.

57,25 g de cet ester et 28,63 g d'amino-4 phtalonitrile sont chauffés à 160°C pendant 7 heures dans 150 cm3 de NMP. La solution est diluée avec 1 litre de dichlorométhane, lavée plusieurs fois à l'eau, séchée et évaporée. Le rendement en (dicyano-3,4 phényl)-N hydroxy-α benzyl-4 phtalimide est de 70 g. Le produit pur obtenu par chromatographie en phase liquide fond vers 150°C.

Exemple 1

Un mélange de 38,83 g de diester de méthyle de l'acide benzhydrol tétracarboxylique-3,3',4,4', 28,65 g d'amino-4 phtalonitrile et 50 g de N-méthylpyrrolidone est chauffé pendant 2 heures à 160-170°C et 0,5 heure à 190°C. La formation des cycles imides s'accompagne de la distillation d'un mélange d'eau et de méthanol (10 g). La solution refroidie est versée dans 500 ml d'eau. Le produit qui précipite est soigneusement lavé à l'eau et séché à 140°C sous vide pendant 20 heures. On obtient avec un rendement de 92 % un solide beige clair dont la température de fusion se situe vers 200-225°C, et dont la formule chimique, déterminée par l'analyse élémentaire et spectroscopique correspond à la formule (2) dans laquelle le radical Ar' est un cycle benzénique substitué sur les atomes de carbone en position 1, 3 et 4.

Exemple 2

Un mélange de 36 g d'acide benzhydrol tétracarboxylique-3,3',4,4' et de 47,1 g de p-aminophénoxy-4 phtalonitrile dans 70 g de N-méthylpyrrolidone est chauffé pendant 2 heures à 150°C, 2 heures à 165°C et 1 heure à 185°C. La formation des cycles imides s'accompagne de la distillation de 7,2 g d'eau. En fin de réaction, la solution est versée dans 600 ml d'eau puis traitée comme dans l'exemple 1. Le diimide obtenu avec un

rendement de 87 % a une température de fusion de 170-180°C et il peut être représenté par la formule (2) dans laquelle le radical Ar' est le groupement (dicyano-3,4 phénoxy)-4 phényle.

Exemple 3

Un mélange de 32,5 g de dianhydride de l'acide benzhydrol tétracarboxylique-3,3',4,4', 50 g de m-aminobenzoyl-4 phtalonitrile, 100 g de méta-crésol et 20 g de benzène est chauffé progressivement jusqu'à 170°C avec distillation azéotropique de l'eau de réaction à l'aide d'un appareil de Dean et Stark. A la fin de la réaction qui dure 4 heures, le benzène et la plus grande partie du méta-crésol sont distillés sous pression réduite. Le résidu visqueux est versé lentement avec une forte agitation dans 500 ml de méthanol. Le précipité est filtré, lavé deux fois avec 100 ml de méthanol bouillant et séché sous vide à 130-140°C pendant 24 heures. Le diimide obtenu avec un rendement de 85 % fond vers 200-207°C et répond à la formule (2) dans laquelle le radical Ar' est le groupement (dicyano-3,4 benzoyl)-3 phényle.

Exemples 4 à 8

Dans un réacteur de 2 litres, on met 650 g de N-méthylpyrrolidone, 466 g de diester de méthyle de l'acide benzhydrol tétracarboxylique-3,3',4,4' et la quantité de bis(amino-4 phényl) méthane indiquée dans le tableau 1. La solution agitée est placée dans un bain d'huile chauffé à 160-170°C pendant 4 heures. On ajoute ensuite la quantité d'amino-4 phtalonitrile indiquée dans le tableau 1 et le chauffage est poursuivi à la même température pendant 3 heures puis à 190-200°C pendant 1 heure. Les produits sont isolés comme dans l'exemple 1 par précipitation dans l'eau.

Les résines de polyimides ainsi préparées ont une masse moléculaire moyenne comprise entre 1000 et 7000 grammes par mole et elles répondent à la formule générale (1) dans laquelle le radical Ar est le diphénylméthane substitué sur les atomes de carbone 4 et 4', le radical Ar' est le benzène substitué sur les atomes de carbone 1, 3 et 4, et le degré de polycondensation n varie de 1 à 19 comme indiqué dans le tableau 1.

Tableau 1

| Exemple N° | Poids en g de Diamine | A.P.N.[1] | $n^{(2)}$ | $\eta_{inh}^{(3)}$ | Masse moléculaire[4] |
|---|---|---|---|---|---|
| 4 | 156,62 | 114,5 | 1 | 0,09 | 1060 |
| 5 | 178,44 | 85,9 | 3 | 0,14 | 2030 |
| 6 | 188,26 | 47,3 | 6 | 0,25 | 3500 |
| 7 | 214,13 | 34,4 | 9 | 0,37 | 4950 |
| 8 | 226,03 | 17,2 | 19 | 0,68 | 9820 |

1) A.P.N. : Amino-4 phtalonitrile

2) n : degré de polycondensation

3) $\eta_{inh}$ : viscosité inhérente

4) masse moléculaire calculée

Les résines de polyimides des exemples 4 et 5 ont une température de ramollissement comprise entre 220 et 280°C et elles peuvent être mises en oeuvre par moulage. Les résines de masse moléculaire plus élevée des exemples 6 à 8 ont un caractère filmogène d'autant plus accentué que la chaîne de polyimide est plus longue. Elles se prêtent donc à la fabrication de revêtements protecteurs. Leur température de transition vitreuse se situe vers 280°C.

Exemples 9 à 14

Dans un réacteur de 1 litre contenant 400 g de méta-crésol et 77,67 g de diester de méthyle de l'acide benzhydrol tétracarboxylique-3,3',4,4', on introduit et on fait réagir en deux étapes, comme dans l'exemple 4, les quantités respectives de bis(amino-4 phényl) éther et d'amino-4 phtalonitrile indiquées dans le tableau 2.

A la fin de la réaction, la solution refroidie à 50-60°C est versée lentement dans 3 litres de méthanol avec une forte agitation à l'aide d'une turbine de broyage. La résine de polyimide qui précipite est lavée plusieurs fois avec 0,5 litre de méthanol bouillant puis séchée à 120°C sous vide pendant 24 heures.

On obtient par ce procédé des résines de polyimides ayant une masse moléculaire moyenne comprise entre 1000 et 10 000 grammes par mole.

Ces résines répondent à la formule générale (1) dans laquelle le radical Ar est le diphényl éther substitué sur les atomes de carbone 4 et 4', le radical Ar' est le benzène substitué sur les atomes de carbone 1, 3 et 4 et dans laquelle le nombre n varie de 1 à 19.

Tableau 2

| Exemple N° | Poids en g de Diamine | Poids en g de A.P.N. | n | $\eta_{inh}$ | Masse moléculaire |
|---|---|---|---|---|---|
| 9 | 20,02 | 28,63 | 1 | 0,08 | 1060 |
| 10 | 30,37 | 14,33 | 3 | 0,15 | 2040 |
| 11 | 33,37 | 9,54 | 5 | 0,20 | 3020 |
| 12 | 35,04 | 7,16 | 7 | 0,28 | 4000 |
| 13 | 36,04 | 5,73 | 9 | 0,38 | 4970 |
| 14 | 38,05 | 2,87 | 19 | 0,70 | 9850 |

Les résines de polyimides des exemples 9 et 10 ont une température de ramollissement comprise entre 215 et 290°C et elles peuvent être mises en oeuvre par moulage. Les résines de masse moléculaire plus élevée des exemples 11 à 14 ont une température de transition vitreuse proche de 280°C et elles conviennent mieux pour la fabrication de revêtements protecteurs.

Exemple 15

Un mélange de 64,85 g de dianhydride de l'acide benzhydrol tétracarboxylique-3,3',4,4', 54,07 g de métaphénylènediamine, 99,13 g de bis(amino-4 phényl) méthane et 28,63 g d'amino-4 phtalonitrile est agité à température ambiante pendant 4 heures puis chauffé pendant 2 heures à 150°C, 2 heures à 170°C et 1 heure à 190°C dans 1 litre de N-méthylpyrrolidone.

La résine obtenue après précipitation dans l'eau avec un rendement de 96 % a une température de ramollissement située vers 220°C.

Exemple 16

Cet exemple est donné à titre comparatif pour mettre en évidence le phénomène d'oxydoréduction intramoléculaire. Les composés modèles des

exemples A et C sont étudiés par analyse thermique différentielle avec un analyseur thermique Mettler programmé pour une montée en température de 10°C/min.

La courbe d'analyse thermique du benzoyl-4 (dicyano-3,4 phényl)-N phtalimide de l'exemple A présente seulement un endotherme de fusion à 250°C. La courbe correspondante du (dicyano-3,4 phényl)-N hydroxybenzyl-4 phtalimide présente un large endotherme de fusion entre 100 et 200°C et un pic exothermique de réaction à 315°C.

Lorsque ces deux produits sont chauffés séparément en atmosphère inerte à 300°C pendant 1 heure, le composé de l'exemple A ne subit pas de transformation importante alors que le composé de l'exemple C s'est transformé en phtalocyanine. Cette transformation est mise en évidence par la disparition totale des bandes d'absorption infrarouge dues aux groupes nitriles à 2230 $cm^{-1}$, l'apparition d'une bande d'absorption de carbonyle cétonique à 1660-1670 $cm^{-1}$ et l'apparition dans le spectre ultraviolet des bandes d'absorption caractéristiques des cycles phtalocyanines.

Exemple 17

Cet exemple est également donné à titre comparatif car il concerne le comportement à la chaleur du bis-(dicyano-3,4 phényl)-N,N' carbonyldiphtalimide-4,4' préparé dans l'exemple 13. Lorsque ce composé est étudié par analyse thermique différentielle, il présente une simple transition endothermique à 260°C correspondant à sa température de fusion. Après un traitement thermique de 30 minutes à 300°C, le composé n'a pas subi de transformation sensible et les spectres infrarouge, ultraviolet et de résonance magnétique nucléaire sont identiques aux spectres du produit de départ.

Exemple 18

Le dérivé du benzhydrol préparé dans l'exemple 1 est étudié par analyse thermique différentielle. La courbe obtenue présente une transition endothermique de fusion qui commence vers 220°C avec un maximum à 250°C. Un pic exothermique de réaction apparaît immédiatement après avec un maximum à 280°C.

Un échantillon de 5 g de composé de l'exemple 1 est placé dans un récipient en verre qui est plongé dans un bain métallique chauffé à

220-230°C. Le produit se ramollit vers 190°C et devient fluide à la température de réaction. Le liquide prend rapidement une couleur verte et sa viscosité augmente après 2 à 3 minutes de chauffage. En moins de 10 minutes, la résine est devenue un solide vert sombre qui ne fond pas à 300°C.

Les spectres infrarouge du produit en cours de polymérisation donnent une indication sur la proportion des groupes nitriles ayant réagi en comparant les intensités respectives de la bande d'absorption de ces groupes à 2230 $cm^{-1}$. Pour une intensité initiale fixée arbitrairement à 100, on obtient les valeurs 60, 55, 44 et 30 après respectivement 1, 2, 3 et 10 minutes de chauffage. La gélification de la résine intervient donc lorsque 60 à 70 % des groupes phtalonitriles se sont transformés en phtalocyanine.

Après la prise en masse de la résine, les réactions de cyano-addition se poursuivent beaucoup plus lentement en phase solide et un traitement de post-cuisson d'une dizaine d'heures vers 280°C est nécessaire pour faire disparaitre la bande d'adsorption due aux groupes nitriles dans le spectre infrarouge.

Lorsque le produit de l'exemple 1 est traité directement à 300°C pendant 1 heure, la bande infrarouge des groupes nitriles a disparu du spectre alors qu'une bande de carbonyle cétonique est présente à 1670 $cm^{-1}$.

L'analyse thermogravimétrique de la résine réticulée de copolyphtalocyanine-imide montre que, en atmsophère inerte, la perte de poids est nulle jusqu'à 300°C. Elle est de 1 % à 400°C et de 10 % à 570°C.

Exemple 19

La résine de polyimide préparée dans l'exemple 7 est mise en solution dans la N-méthylpyrrolidone de façon à avoir une concentration en matière sèche de 40 %. Cette solution est utilisée pour imprégner du tissu de verre E-181 traité avec un apprêt au γ-aminopropyltriéthoxysilane. L'imprégnation du tissu de verre est réalisée sur les

deux faces de façon à obtenir après évaporation du solvant un tissu préimprégné contenant 35 % de résine et 65 % de tissu de verre. Ce tissu, séché à 140°C dans une étuve à ventilation forcée, est ensuite découpé en 16 éléments identiques de 20 x 20 cm.

Ces éléments sont superposés et sont placés entre les deux plateaux d'une presse hydraulique chauffés à 250°C. Après un temps de contact de 5 minutes, on applique une pression de 20 bars et la température est portée à 300°C en 20 minutes. Le traitement sous pression à 300°C dure 2 heures. Après refroidissement sous pression, on obtient un matériau stratifié dense de couleur vert sombre ayant un pourcentage de vide inférieur à 1 %. L'analyse thermogravimétrique de ce matériau indique qu'en atmosphère inerte, la décomposition de la matrice organique commence vers 500°C, et devient assez rapide à 550°C. Le matériau ne perd pas de poids après 1000 heures à 250°C et perd seulement 10 % de son poids après 1000 heures à 300°C.

Exemple 20

Dans un broyeur à boulets, on réalise un mélange intime de résine de l'exemple 13 (50 g) et de résine de l'exemple 1 (50 g). Ce mélange est placé dans un moule cylindrique de 10 cm de diamètre qui est chauffé à 250°C avec une pression de 10 bars . Après 10 minutes à cette température, le matériau est chauffé pendant 2 heures à 300°C. Au refroidissement, on obtient un disque homogène et dense de couleur vert sombre de polyimide réticulé. Après un recuit de 24 heures à 280°C, ce matériau moulé présente une excellente stabilité thermique et il est utilisable en service de longue durée entre 250 et 300°C.

Exemple 21

Une solution à 20 % en poids dans le méta-crésol de la composition de polyimides de l'exemple 13 est étendue sur une plaque de cuivre avec un filmographe de 150 micromètres. Le solvant est évaporé dans une étuve à ventilation forcée à 80, 100, 120 et 150°C pendant 15 minutes à chacune de ces températures. Le revêtement est ensuite durci pendant 1 heure à 200, 250, 280 et 300°C pour donner un vernis de couleur verte qui adhère fortement sur le métal. Ce vernis a une bonne souplesse et une grande résistance à la rayure. Sa température de thermoplasticité est supérieure à 350°C.

## Revendications

1/ Composition de résines de polyimides aromatiques, caractérisée en ce qu'elle est le produit pouvant être obtenu par une mise en réaction entre 80 et 300°C d'au moins un composé aromatique (A) de formule générale :

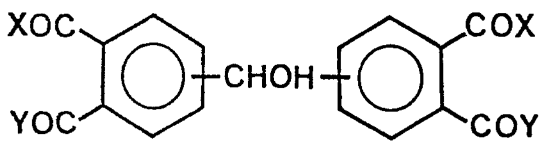

dans laquelle X et Y sont, indépendamment, choisis parmi les restes hydroxyles et hydroxycarbyloxy, ou forment ensemble un atome d'oxygène ;

avec au moins un amino-dinitrile aromatique (C) de formule générale :

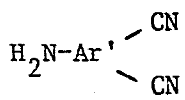

dans laquelle Ar' est un radical aromatique trivalent carbocyclique ou hétérocyclique dont les trois valences sont sur des atomes de carbone distincts, deux d'entre eux, ceux qui portent les groupes nitriles, étant situés en position ortho l'un par rapport à l'autre ; et facultativement avec au moins une diamine aromatique (B) de formule générale :

$$H_2N\text{-}Ar\text{-}NH_2$$

dans laquelle Ar est un radical aromatique divalent carbocyclique ou hétérocyclique dont les deux valences sont situées sur des atomes de carbone distincts non situés en position ortho l'un par rapport à l'autre.

2/ Composition selon la revendication 1, dans la préparation de laquelle on utilise, pour chaque centre réactif ortho-difonctionnel du composé(A), de 0,9 à 1,1 fonction amine primaire provenant du composé (C) ou éventuellement de l'ensemble des composés (B) et (C).

3/ Composition selon la revendication 1, dans la préparation de laquelle on utilise, pour chaque centre réactif ortho-difonctionnel du composé (A), de 0,98 à 1,02 fonction amine primaire provenant du composé (C) ou

éventuellement de l'ensemble des composés (B) et (C)

4/ Composition selon l'une des revendications 1 à 3, dans la préparation de laquelle les réactifs comprennent seulement au moins un composé (A) et au moins un composé (C), et on utilise au moins 2 moles de composé (C) par mole de composé (A)

5/ Composition selon l'une des revendications 1 à 3, dans la préparation de laquelle on utilise, pour une mole de composé (A), respectivement de 0 à 0,98 mole de composé (B) et 2 à 0,04 mole de composé (C).

6/ Composition selon l'une des revendications 1 à 5, dont la réaction d'obtention comprend d'abord la réaction du composé (A) avec la diamine (B), en utilisant un excès de composé (A) par rapport à la proportion stoechiométrique d'un centre réactif du composé (A) par fonction amine primaire du composé (B) jusqu'à obtention d'un produit (D) d'une masse moléculaire moyenne comprise entre 800 et 25 000 , puis la réaction du produit (D) avec l'amino-dinitrile (C) utilisé en proportion suffisante pour avoir un nombre total de fonctions amines primaires des composés (B) et (C) représentant 0,9 à 1,1 fois le nombre de centres réactifs ortho-difonctionnels du composé (A).

7/ Composition selon l'une des revendications 1 à 6, dans la préparation de laquelle on fait réagir un diacide, anhydride, diester ou acide-ester benzhydrol tétracarboxylique-3,3',4,4' sur l'amino-4 phtalonitrile, le p-aminophénoxy-4 phtalonitrile ou le m.aminobenzoyl-4 phtalonitrile.

8/ Composition selon la revendication 6, dans la préparation de laquelle on fait réagir un diacide, anhydride, diester ou acide-ester benzhydrol tétracarboxylique-3,3',4,4' sur le bis(amino-4 phényl) méthane ou le bis(amino-4 phényl) éther puis sur l'amino-4 phtalonitrile.

9/ Composition selon l'une des revendications 1 à 6 dans la préparation de laquelle la température est de 100 à 200° C.

10/ Composition selon l'une des revendications 1 à 9, dans la préparation de laquelle la réaction est interrompue avant gélification substantielle de la résine.

11/ Le produit de réticulation d'une composition selon l'une des revendications 1 à 10, ledit produit étant obtenu par chauffage d'une telle composition et étant caractérisé par la présence de cycles phtalocyanine.

Office européen des brevets

**0170590**
Numéro de la demande

# RAPPORT DE RECHERCHE EUROPEENNE

EP 85 40 1501

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | EP-A-0 077 718 (I.F.P.)<br>--- | | C 07 D 209/48<br>C 08 G 73/10<br>C 09 D 3/49<br>C 09 J 3/16 |
| A | US-A-3 939 109 (W. BAROC)<br>----- | | |
| | | | **DOMAINES TECHNIQUES RECHERCHES (Int. Cl.4)** |
| | | | C 07 D<br>C 08 G |

Le présent rapport de recherche a été établi pour toutes les revendications

| Lieu de la recherche | Date d'achèvement de la recherche | Examinateur |
|---|---|---|
| LA HAYE | 15-10-1985 | LEROY ALAIN |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant